(19) Europäisches Patentamt

European Patent Office

Office européen des brevets

(11) EP 0 749 755 A2

## (12) DEMANDE DE BREVET EUROPEEN

(43) Date de publication:
27.12.1996 Bulletin 1996/52

(51) Int Cl.6: **A61K 49/00**

(21) Numéro de dépôt: 96401170.4

(22) Date de dépôt: 31.05.1996

(84) Etats contractants désignés:
CH DE FR GB LI

(30) Priorité: 19.06.1995 FR 9507302

(71) Demandeur: CENTRE INTERNATIONAL DE RECHERCHES DERMATOLOGIQUES GALDERMA (C.I.R.D. GALDERMA) F-06560 Valbonne (FR)

(72) Inventeurs:
• Demarchez, Michel
06620 Le Bar sur Loup (FR)

• Jomard, André
06460 Saint Vallier de Thiey (FR)

(74) Mandataire: Andral, Christophe André Louis L'OREAL
Centre de Recherche Charles Zviak
Département Propriété Industrielle
90, rue du Général Roguet
92583 Clichy Cedex (FR)

(54) **Procédé pour identifier des composés antagonistes des récepteurs RARs**

(57) La présente invention concerne un procédé pour identifier des molécules antagonistes des RARs, caractérisé en ce qu'il comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère.

## Description

La présente invention a pour objet un procédé pour identifier des composés antagonistes des récepteurs RARs en utilisant un mammifère, tel que les rongeurs (rat, cobaye, hamster, lapin, souris...).

On sait que l'acide rétinoïque *tout-trans* est un puissant modulateur (i.e. un inhibiteur ou, au contraire, un stimulateur, selon la nature des cellules traitées) de la différenciation et de la prolifération de nombreux types cellulaires normaux ou transformés. Par exemple, il inhibe la différenciation des cellules épithéliales, tels que les kératinocytes de l'épiderme. Il inhibe aussi la prolifération de nombreuses cellules transformées telles que les cellules de mélanomes.

On sait, d'une manière générale, que l'acide rétinoïque *tout-trans (all-trans* retinoic acid) agit sur la différenciation et la prolifération des cellules en interagissant avec des récepteurs nucléaires appelés RARs (Retinoic Acid Receptors) contenus dans le noyau cellulaire. Ces récepteurs, aprés fixation du ligand (i.e. de l'acide rétinoïque *tout-trans),* interagissent avec la région promotrice de gènes régulés par l'acide rétinoïque au niveau d'éléments de réponses spécifiques. Pour se fixer sur les éléments de réponse, les RARs s'hétérodimèrisent avec un autre type de récepteurs connus sous le nom de RXRs, le ligand naturel des RXRs étant l'acide 9-*cis*-rétinoïque.

De nombreux analogues structuraux synthétiques de l'acide rétinoïque *tout-trans* ou de l'acide 9-*cis*-rétinoïque, couramment dénommés "rétinoïdes", ont été décrits à ce jour dans la littérature. Certaines de ces molécules sont capables de se fixer et d'activer (agonistes) ou à l'inverse de désactiver (antagonistes) spécifiquement les RARs. Par exemple, l'acide rétinoïque *tout-trans* active sélectivement les RARs et ainsi est considéré comme une molécule agoniste des RARs.

Chez la souris, il a été montré que l'acide rétinoïque *tout-trans* en administration unique par voie topique induit une réponse, dépendante de la dose, de prolifération de l'épiderme, avec un maximum de réponse quatre jours après l'application ("Retinoïc acid provokes a regeneration-like proliferative response in murine epidermis" Arch. Dermatol. Res. 1992, 284:418-423). En accord avec ce résultat, la réponse à une application topique d'acide rétinoïque *tout-trans* sur l'oreille de souris se traduit notamment par une augmentation de l'épaisseur de l'oreille de souris. Cette augmentation de l'épaisseur de l'oreille de souris semble être due à une augmentation de l'épaisseur de l'épiderme et à une apparition d'un oedème dermique. Cette réponse peut donc être facilement mesurée à l'aide d'un appareil, tel que l'oditest, celle-ci étant maximale aux cinquième et sixième jours après l'application. Cette réponse se vérifie avec toutes les molécules agonistes des RARs.

En ce qui concerne les antagonistes des RARs, ceux-ci s'avèrent notamment intéressants dans le traitement de désordres ou des affections dermatologiques, rhumatismales, respiratoires, cardiovasculaires, osseuses ou encore ophtalmologiques notamment liées à une surrégulation (surexpression ou suractivité) des récepteurs RARs et/ou à une hypervitaminose A (présence dans l'organisme d'une quantité anormale de vitamine A ou de ses métabolites). Ainsi, on comprend l'intérêt de trouver de nouveaux composés antagonistes RARs qui peuvent venir inhiber les effets biologiques d'une surrégulation des récepteurs RARs et/ou d'une hypervitaminose A.

La Demanderesse vient de découvrir que la réponse à une application topique sur la peau d'un mammifère d'une molécule agoniste des RARs peut être inhibée par l'administration par voie systémique ou topique d'une molécule antagoniste des RARs.

Ainsi, la présente invention a pour but de proposer un procédé simple pour identifier des molécules antagonistes des RARs.

Ce but et d'autres sont atteints par la présente invention qui concerne un procédé pour identifier des molécules antagonistes des RARs, caractérisé en ce qu'il comprend les étapes suivantes : (i) on applique topiquement sur une partie de la peau d'un mammifère une quantité suffisante d'au moins une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse de la partie de la peau ainsi traitée du mammifère et on la compare avec la réponse obtenue sur cette même partie de peau traitée par la seule étape (i).

Ainsi, lorsque la molécule administrée est un antagoniste des RARs, l'augmentation de l'épaisseur de la partie de la peau traitée du mammifère par une molécule agoniste des RARs n'est pas constatée ou est diminuée. On observe donc une inhibition de la réponse.

De manière pratique, le mammifère est un rongeur tel qu'une souris, un rat, un cobaye, un hamster ou un lapin.

La partie de la peau de mammifère utilisée peut être n'importe quelle partie du corps du mammifère.

La réponse sur la partie de la peau ainsi traitée du mammifère à évaluer correspond à une modification clinique de celle-ci. De manière générale, cette réponse à évaluer correspond à une modification de l'épaisseur de la partie de la peau ainsi traitée.

Ainsi, la mesure de l'épaisseur de la partie de la peau ainsi traitée peut être réalisée par toute méthode connue en soi.

Lorsque la partie de la peau utilisée est lisse, on peut mesurer son épaisseur en la pliant.

De manière plus pratique, on utilise la peau de l'oreille. La mesure de l'épaisseur de l'oreille peut alors être réalisée par un oditest.

Bien entendu, l'évaluation de l'étape (iii) correspond à une mesure de la réponse de la partie de la peau ainsi traitée et à une comparaison de cette mesure avec celle de la réponse de cette même partie de la peau traitée, dans les mêmes conditions, avec la molécule agoniste des RARs seule.

De préférence, les molécules agonistes des RARs sont choisis parmi les composés capables d'induire la différenciation des cellules (F9) du tératocarcinome embryonnaire de souris. La sécrétion de l'activateur plasminogène qui accompagne cette différenciation est un indice de la réponse biologique des cellules F9 à ces composés. On sait également que la capacité de ces composés à induire l'activateur plasminogène est corrélé directement avec l'affinité et l'activité qu'ils ont sur les récepteurs RARs endogènes aux cellules F9 (Skin Pharmacol., 1990, 3, pp. 256-267).

Parmi les molécules agonistes des RARs induisant la différenciation des cellules F9, on peut plus particulièrement citer :

- l'acide rétinoïque *tout-trans,*
- l'acide 2-(5,6,7,8-tetrahydro-5,5,8,8-tetraméthyl-2-naphtyl)-6-benzo[b]thiophen carboxylique,
- l'acide 4-[(5,6,7,8-tétrahydro-5,5,8,8-tétraméthyl-2-naphtyl) carboxamido] benzoïque,
- l'acide 4-[(5,6,7,8-tétrahydro-5,5,8,8-tétraméthyl-2-naphtyl) carbamoyl] benzoïque.

Dans ce qui suit ou ce qui précède, on entend par voie topique, toute technique d'administration d'un produit par application directe de ce dernier sur une partie superficielle (ou externe) du corps, et par voie systémique, toute technique d'administration d'un produit par une voie autre que topique, par exemple entérale et/ou parentérale. Dans le cas de la voie systémique, on préfère utiliser la voie orale.

La quantité suffisante d'une molécule agoniste des RARs à appliquer correspond à celle à laquelle on observe une réponse de la partie traitée de la peau du mammifère après l'étape (i). Ainsi, de préférence et selon la nature de la molécule agoniste des RARs utilisée, cette quantité varie entre 0,0001% et 2% en poids par volume de solution appliquée.

On va maintenant donner, à titre nullement limitatif, plusieurs exemples.

## EXEMPLE 1

Le test utilisé est donc celui de l'oedème de l'oreille de souris induit par application topique de l'acide 2-(5,6,7,8-tetrahydro-5,5,8,8-tetraméthyl-2-naphtyl)-6-benzo[b]thiophen carboxylique (analogue de l'acide rétinoïque *tout-trans)* à 0,01% en poids par volume. Selon ce modèle, une application topique de l'acide 2-(5,6,7,8-tetrahydro-5,5,8,8-tetraméthyl-2naphtyl)-6-benzo[b]thiophen carboxylique sur l'oreille provoque une inflammation qui se caractérise par une augmentation de l'épaisseur de l'oreille de souris, cette augmentation devenant maximal au bout de 5 jours après l'application. Cette réponse peut donc être quantifiée par une mesure de l'épaisseur de l'oreille par un oditest.

Le protocole opératoire exact est le suivant : 10 souris sont tout d'abord traitées avec l'acide 2-(5,6,7,8-tetrahydro-5,5,8, 8-tetraméthyl-2-naphtyl)-6-benzo[b]thiophen carboxylique (composé A), en procédant sur l'une de leur oreille à une application topique à un temps t=0 avec 20 μl d'une solution d'acétone comprenant 0,01 % en poids par volume d'acide carboxylique 2-(5,6,7,8-tetrahydro-5,5,8,8-tetraméthyl-2naphtyl)-6-benzo[b]thiophen. Simultanément, on applique en une fois par voie topique, sur 5 (= groupe 2) des 10 souris traitées par le composé A, 0,1 % en poids par volume de l'acide *trans* 7-[3-(1-adamantyl)-4-méthoxyphenyl]-3,7-diméthyl-2,4,6-heptatrienoïque (composé X) dans de l'acétone à partir de t=0. Les 5 souris n'ayant pas été traitées par le composé X constituent le groupe 1. La réponse est quantifiée par une mesure de l'épaisseur de l'oreille à t = 5 ou 6 jours. Les résultats sont ensuite exprimés en % d'inhibition calculé de la façon suivante :

$$\frac{\text{épaisseur oreille souris (Groupe 1) - épaisseur oreille souris (Groupe 2)}}{\text{épaisseur oreille souris (Groupe 1)}} \times 100$$

Le composé X se révèle par un autre test comme étant un antagoniste des RARs. En effet, l'activité antagoniste du composé X est évaluée dans le test de différenciation des cellules F9 de tératocarcinome embryonnaire de souris (Cancer Research 43, p 5268, 1983). Ce composé testé à $10^{-6}$ M est inactif en tant qu'agoniste dans ce test et inhibe partiellement ou totalement l'effet produit par un rétinoide agoniste sur la morphologie et sur la sécrétion de l'activateur du plasminogène (la sécrétion de l'activateur du plasminogène qui accompagne la différenciation des cellules F9 étant un indice de la réponse biologique des celules F9 aux rétinoïdes).

Les résultats obtenus sont rassemblés dans le tableau 1 suivant.

tableau 1

| voie topique | dose (%P/V) | voie topique | dose (%P/V) | Inhibition (%) |
|---|---|---|---|---|
| composé A | 0,01 | composé X | 0,1 | 58,60 |
| P/V signifie poids par volume. | | | | |

Ainsi, on démontre clairement, grâce à ce test, que l'association d'un agoniste de récepteurs RARs avec un antagoniste des récepteurs RARs inhibe de manière importante la réponse de l'oreille de souris comparativement à la réponse induite par une application topique unique d'un ligand de récepteurs RARs.

EXEMPLE 2

On réalise exactement le même test qu'à l'exemple 1, sauf que le composé A est remplacé par l'acide rétinoïque *tout-trans* (composé B).

Les résultats obtenus sont rassemblés dans le tableau 2 suivant.

tableau 2

| voie topique | dose (%P/V) | voie topique | dose (%P/V) | Inhibition (%) |
|---|---|---|---|---|
| composé B | 0,1 | composé X | 0,1 | 32 |
| P/V signifie poids par volume. | | | | |

EXEMPLE 3

On réalise exactement le même test qu'à l'exemple 1, sauf que le composé X est remplacé par un composé Y : Acide 4-[3-(1-adamantyl)-4-methoxyethoxymethoxyphenylethynyl]benzoïque ou par un composé Z : Acide 4-[7-(1-adamantyl)-6-méthoxyéthoxyméthoxy-2-naphtyl]benzoïque.

Les composés Y et Z se révèlent par ailleurs comme étant des antagonistes des RARs, l'activité antagoniste des composés Y et Z ayant été évaluée dans le test de différenciation des cellules F9 de tératocarcinome embryonnaire de souris, tel que décrit dans l'exemple 1.

Les résultats obtenus sont rassemblés dans le tableau 3 suivant.

tableau 3

| voie topique | dose (%P/V) | voie topique | dose (%P/V) | Inhibition (%) |
|---|---|---|---|---|
| composé A | 0,01 | composé Y | 0,1 | 81 |
| composé A | 0,01 | composé Z | 0,1 | 92 |
| P/V signifie poids par volume. | | | | |

EXEMPLE 4

On réalise exactement le même test qu'à l'exemple 1, sauf que le composé A est remplacé par le composé B (tel que décrit à l'exemple 2) et le composé X est remplacé par les composés Y ou Z (tels que décrits à l'exemple 3).

Les résultats obtenus sont rassemblés dans le tableau 4 suivant :

tableau 4

| voie topique | dose (%P/V) | voie topique | dose (%P/V) | Inhibition (%) |
|---|---|---|---|---|
| composé B | 0,1 | composé Y | 0,1 | 32 |
| composé B | 0,1 | composé Z | 0,1 | 86 |

EXEMPLE 5

On réalise exactement le même test qu'à l'exemple 1, sauf que les composés Z et W, remplaçant le composé X, sont administrés par voie orale dans de l'huile de type cremophor (EL 25%), au lieu d'être appliqués par voie topique, aux doses indiquées dans le tableau 5 ci-dessous.

Le composé W est : Acide p-[(E)-2-[3',4'-dihydro-4',4'-diméthyl-7'-(heptyloxy)-2'H-1-benzothiopyran-6'-yl]propenyl]-benzoïque

Le composé W se révèle par ailleurs comme étant un antagoniste des RARs, l'activité antagoniste de ce composé ayant été évaluée dans le test de différenciation des cellules F9 de tératocarcinome embryonnaire de souris, tel que décrit dans l'exemple 1.

Les résultats obtenus sont rassemblés dans le tableau 5 suivant :

tableau 5

| voie topique | dose (%P/V) | voie orale | dose (%P/V) | Inhibition (%) |
|---|---|---|---|---|
| composé A | 0,01 | composé Z | 30 | 45 |
| composé A | 0,01 | composé W | 30 | 79 |

**Revendications**

1. Procédé pour identifier des molécules antagonistes des RARs, caractérisé en ce qu'il comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse de la partie de la peau ainsi traitée du mammifère et on la compare avec la réponse obtenue sur cette même partie de peau traitée par la seule étape (i).

2. Procédé selon la revendication précédente, caractérisé en ce que le mammifère est un rongeur tel qu'une souris, un rat, un cobaye, un hamster ou un lapin.

3. Procédé selon l'une des revendications précédentes, caractérisé en ce que la partie de la peau de mammifère utilisée est la peau de l'oreille.

4. Procédé selon l'une des revendications précédentes, caractérisé en ce que la réponse à évaluer à l'étape (iii) correspond à une modification de l'épaisseur de la partie de la peau ainsi traitée du mammifère.

5. Procédé selon l'une quelconque des revendications précédentes, caractérisé en ce que la molécule agoniste des RARs est choisie parmi les composés capables d'induire la différenciation des cellules (F9) du tératocarcinome embrionnaire de souris.

6. Procédé selon la revendication précédente, caractérisé en ce que la molécule agoniste des RARs utilisée est choisie parmi :

   - l'acide rétinoïque *tout-trans,*
   - l'acide 2-(5,6,7,8-tetrahydro-5,5,8,8-tetraméthyl-2-naphtyl)-6-benzo[b]thiophen carboxylique,
   - l'acide 4-[(5,6,7,8-tétrahydro-5,5,8,8-tétraméthyl-2-naphtyl) carboxamido] benzoïque,
   - l'acide 4-[(5,6,7,8-tétrahydro-5,5,8,8-tétraméthyl-2-naphtyl) carbamoyl] benzoïque.

7. Procédé selon l'une quelconque des revendications précédentes, caractérisé en ce que la voie systémique est la voie orale.